# EUROPEAN PATENT SPECIFICATION

(11) **EP 4 606 312 B1**
(45) Date of publication and mention of the grant of the patent: **05.08.2026**
(21) Application number: 24158633.8
(22) Date of filing: 20.02.2024
(51) Int. Cl.: A61B 6/50, A61B 6/51, A61B 6/00, A61B 6/58

(54) **METHOD OF GENERATING A CEPHALOMETRIC IMAGE WITH REDUCED CHATTER MARKS OF A PATIENT'S HEAD**
VERFAHREN ZUR ERZEUGUNG EINES ZEPHALOMETRISCHEN BILDES MIT REDUZIERTEN RATTERMARKEN DES KOPFES EINES PATIENTEN
PROCÉDÉ DE GÉNÉRATION D'UNE IMAGE CÉPHALOMÉTRIQUE AVEC DES MARQUES DE BROUTEMENT RÉDUITES DE LA TÊTE D'UN PATIENT

(43) Date of publication of application: 27.08.2025
(73) Proprietor: DENTSPLY SIRONA Inc., York, PA 17401 (US); SIRONA Dental Systems GmbH, 64625 Bensheim (DE)
(72) Inventor: EICHNER, Stefan, 64625 Bensheim (DE)
(74) Representative: Aldridge, Henry Alexander

(56) References cited:
- US-A- 5 359 637
- US-A1- 2015 305 696
- US-A1- 2016 220 212
- US-A1- 2019 254 617

## Description

### TECHNICAL FIELD OF THE INVENTION

The present invention relates to a computer-assisted extraoral x-ray system and a computer-implemented method of generating a cephalometric image of a patient's head.

### BACKGROUND ART OF THE INVENTION

A cephalometric (ceph) image (cephalogram) is part of an orthodontist's standard diagnostics. Ceph images are required, among other things, to determine relevant positions in the patient's jaw for the planning, implementation, and follow-up of an orthodontic treatment.

Various digital ceph systems exist in today's dental market for extraoral x-ray systems.

On the one hand, there is the so-called one-shot ceph, whereby a correspondingly large, expensive digital ceph sensor is used to capture the patient's head directly with only one - short static exposure. This prevents motion artefacts, for example, due to the reduced exposure time, but such ceph sensors are often expensive and bulky. Such a one-shot ceph system is not part of this application.

On the other hand, linear scan systems are another option for creating a ceph image. Here, the patient is sequentially scanned at the required focus-detector distance (usually approx. >1.5 m). In addition to a primary aperture in the x-ray source, an additional secondary aperture associated with the ceph detector is commonly used for additional x-ray dose reduction. The exposure time is longer than with one-shot ceph, but the ceph sensor costs (i.e., a digital ceph sensor based on CCD/CMOS ) are significantly lower. Due to the sequential recording technology, however, the demands on the x-ray device (mechanics, motors) are significantly higher, as the ceph sensor used must be moved synchronously with the secondary aperture along the patient's head during the recording.

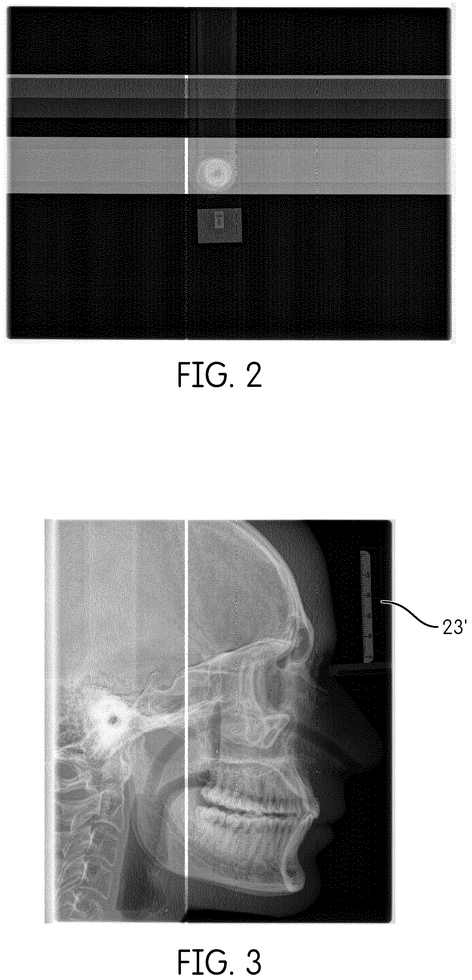

Older linear scan systems based on this approach use CCD-based ceph sensors according to the commonly known TDI principle (i.e., time delay integration). This means that the summation of the image information takes place directly in the ceph sensor and one image column of the ceph image to be created is always output sequentially by such a ceph sensor. The integration time and readout speed are determined by the TDI frequency. However, a subsequent analysis of the image information per acquisition time through software is not possible with this TDI-based linear scan system variant. With the TDI method, all individual image information is generally processed directly in the ceph sensor. In general, mechanical and motor-related deviations from the target behavior of the linear scan system during operation can lead to disturbances in the acquisition process, which in turn cause image artifacts, including so-called chatter marks in the ceph image. These reduce the image quality and diagnostic suitability (i.e., risk of misdiagnosis) of the ceph images and may lead to repeat exposure. When the secondary aperture and the ceph detector are not synchronously aligned during the linear scan (e.g., when the center of the secondary aperture is not synchronously coincided with the center of the exposed frame being read out), the grey level peak of the distributions of the image data ( i.e., generally a gaussian-like distribution across the secondary aperture) captured by the ceph sensor are relatively shifted from their ideal centered positions on the ceph detector, which in turn leads during the add & shift reconstruction of the ceph image to grey-level intensity variations which are conspicuous in form of vertical and/or horizontal stripes. Thus, the frame being read out must be uniformly exposed. Examples of chatter marks can be seen in the ceph image 23' in Fig. 3 by clear non-patient-specific fluctuations in the intensity of the gray values. Especially in so-called technical ceph images (i.e., a ceph image without a patient), chatter marks can also be seen as shown in Fig. 2 due to clear fluctuations in the intensity of the gray values.

The above-mentioned one-shot sensors for ceph imaging solve the chatter mark problem completely but are very expensive and not possible on every extraoral x-ray system. Some extraoral x-ray systems with linear scan ceph technology completely dispense with the secondary aperture to avoid the chatter mark problem, however which can lead to a higher dose and thus to unnecessary patient hazard. With liner-scan ceph technology without a secondary aperture, the scan speed can be increased to keep the dose constant, but this can lead to a reduction in image quality.

US2015305696A1 may be considered to disclose may be considered to disclose a computer-implemented method of generating a cephalometric image of a patient's head, comprising: a step of acquiring a sequence of projection images by exposing the patient's head with x-rays from an x-ray source while linearly moving an x-ray ceph detector and an x-ray ceph collimator on opposing sides along the patient's head, wherein the x-ray ceph collimator has an aperture and one or more x-ray opaque markers which can be detected in the projection images, and wherein the projection images are sequentially read out at a predetermined rate from a predetermined frame of the x-ray ceph detector; a step of detecting the real position of the at least one x-ray opaque marker in the projection images relative to a target position on the ceph detector; a shift and add reconstruction step of adding the projection images after shifting projection images by a predetermined shift amount based on the predetermined rate; wherein the positions of the shifted projection images are adjusted by an amount corresponding to a difference in the real position and the target position of the least one marker detected in the corresponding projection image to reduce chatter marks prior to the adding in the shift and add reconstruction step.

### DISCLOSURE OF THE INVENTION

The inventor is not aware of any prior art technology in which the real position of the gaussian like grey-level distribution of the projection image data are adjusted to the ideal positions during the reconstruction of the ceph image by the shift & add technique to reduce the effect of chatter marks, by using opaque markers placed in the ceph aperture to allow the detection of the level of adjustment required due to the incorrect positioning/alignment of the ceph aperture with respect to the ceph detector.

An objective of the present invention is to provide an extraoral x-ray system and a computer-implemented method of generating a cephalometric image of a patient's head in which chatter marks are educed.

This objective is achieved by the computer-implemented method as defined in claim 1, and the extraoral x-ray system as defined in claim 6. The subject-matters of the dependent claims define further developments and preferred embodiments.

The method according to the present invention is a computer-implemented method which can be realized as a software, namely by computer readable codes, to be executed in conjunction with an extraoral x ray system. The computer-implemented method is for generating a cephalometric image of a patient's head. The method comprises: a step of acquiring a sequence of projection images by exposing the patient's head with x-rays from an x-ray source while linearly moving an x-ray ceph detector and an x-ray ceph collimator on opposing sides along the patient's head, wherein the x-ray ceph collimator has an aperture and one or more x-ray opaque markers which can be detected in the projection images, and wherein the projection images are sequentially read out at a predetermined rate from a predetermined frame of the x-ray ceph detector; a step of detecting the real position of the at least one x-ray opaque marker in each of the projection images relative to a target position on the ceph detector; a shift & add reconstruction step of adding the projection images after shifting each projection image relative to its preceding projection image by a predetermined shift amount based on the predetermined rate; wherein the positions of the shifted projection images are individually adjusted by an amount corresponding to a difference in the real position and the target position of the least one marker detected in the corresponding projection image to reduce chatter marks prior to the adding. In this context, the real position refers to the measured position, whereas the target position refers to the ideal would-be position at which the ceph aperture would have been symmetrically positioned/aligned with the read out frame of the ceph sensor. The target positions can be ideally predetermined through the geometry of the x-ray system.

According to the present invention, the marker can be implemented in various alternative different ways, and used alone or in combination. According to an embodiment of the present invention, the marker is defined through the ceph collimator edge surrounding the ceph aperture. In other alternative embodiments of the present invention, the marker comprises one or more recesses and/or protrusions formed on the ceph collimator edge surrounding the ceph aperture. In other alternative embodiments of the present invention, the marker comprises one or more holes formed in the vicinity of the ceph collimator edge surrounding the ceph aperture.

Generally, the chatter marks are image artefacts which spatially extend in vertical directions. However, they may also be inclined due to tilting disturbances in the mechanics. The present invention also optionally deals with the reduction of chatter marks which are inclined. Therefore, in an embodiment of the present invention, in the detecting step, the real position of at least one additional (second) x-ray opaque marker is detected in each of the projection images in order to determine the alignment to be adjusted, wherein the position and alignment of the shifted projection images are individually adjusted in accordance with two marker detected in the corresponding projection image prior to the adding.

The present invention also provides an extraoral x-ray system comprising: an x-ray source; an x-ray ceph detector; and an x-ray ceph collimator, wherein the x-ray ceph collimator has at least one x-ray opaque marker; a control means for controlling the x-ray source, the x-ray ceph detector and the x-ray ceph collimator, wherein the control means is configured to execute the method according to any one of the preceding claims. The extraoral x-ray system can be provide solely for ceph imaging. Alternatively, it can be provided in combination with PAN and/or DVT imaging.

The computer-implemented method is provided as a computer program, namely a software comprising computer-readable code which, when executed by the computer-assisted extraoral x-ray system, causes said system to perform the method of the present invention.

An important advantageous effect of the present invention is that it improves the diagnostic capability and image quality of ceph images on extraoral x-ray systems using linear scan technology. This is achieved by using a frame based ceph sensor, i.e., a ceph sensor wherein the x-ray projection images are sequentially read out, including subsequent reconstruction of the ceph image by means of a software. The frame-based ceph sensor reads out a large number of projection images during the ceph scan. Since the individual projection images are made available to the software during and after the ceph acquisition, the system-related disturbances (the cause of image quality artifacts) can be subsequently reduced by spatially adjusting the individual projections images based on detection of the markers real positions. A deviation of the marker real position from the target (ideal) position provides a measure for the spatial adjustment of the image data of the projection images. Overall, the present invention significantly improves the determination of relevant positions in the patient's head for the planning, implementation, and follow-up of orthodontic treatments.

A further important advantageous effect of the present invention is that since these image artifacts, namely the chatter marks can be significantly reduced by using a software, thereby no higher demands are placed on the mechanics, motors of the extraoral x-ray system with the linear scan ceph technology. By reconstructing the ceph image using the software, the image quality and diagnostic suitability of the ceph images can be significantly increased and repeat exposures can be avoided.

A further important advantageous effect of the present invention is that it allows reliable use of frame-based ceph sensors, which are less expensive and much smaller in size than one-shot ceph sensors. Thus, the present invention does not place any increased demands on the mechanics and electronics of an x-ray ceph system with linear scan technology, and enables a reduction of chatter marks through the dedicated software.

A further important advantageous effect of the present invention is that it reliably allows the use of a ceph collimator for additional dose reduction for the patient during the ceph acquisition, while also reducing chatter marks.

The reduction of chatter marks is achieved by analyzing and adjusting the projection images through the software before the final reconstruction, which in turn leads to improved image quality. Thereby, the projections images can also be preprocessed prior to the reconstruction for dark current, gain, pixel defects and the like.

The data volume of a frame-based ceph image (projection images) is significantly higher compared to a TDI-based linear scan system. Thus, high quality ceph images can be generated with reduced chatter marks.

In sum, the method according to the present invention creates diagnostic and therapeutic added values for the dentist through more reliable diagnosis due to the chatter marks reduced ceph images.

### BRIEF DESCRIPTION OF THE DRAWINGS

In the following description, the present invention will be explained in more detail with reference to the exemplary embodiments and with reference to the drawings, wherein.
- Fig. 1 -: shows a schematic drawing of an extraoral x-ray system according to an embodiment of the present invention;
- Fig. 2 -: shows a ceph image taken without a patient (i.e., a so called technical ceph image) having chatter marks, and acquired with an extraoral x-ray system according to the prior art;
- Fig. 3 -: shows a ceph image of a patient's head, having chatter marks, and acquired with an extraoral x-ray system according to the prior art;
- Fig. 4A-4C -: show various schematic drawings illustrating the relative positions of the x-ray ceph detector, specifically a predetermined frame (area) thereof, and the ceph collimator edge according to an embodiment of the invention, wherein the Fig.4A shows an ideal symmetrical arrangement, the Fig. 4B shows an incorrect arrangement, and the Fig. 4C shows a displacement arrow for adjusting the image information due to the incorrect arrangement in Fig. 4B;
- Fig. 5A-5C -: shows various schematic drawings of a multi-component ceph collimator having various markers (protrusion, recess, hole, edge) according to alternative embodiments of the present invention, wherein the two sided arrows show the movable components;
- Fig. 6A-6C -: shows various schematic drawings of a single piece ceph collimator having various markers (protrusion, recess, hole, edge) according to further alternative embodiments of the present invention;
- Fig. 7A: shows a comparative example for a ceph image of a phantom head, having chatter marks which have not be reduced;
- Fig. 7B: shows a ceph image of a phantom head, having reduced chatter marks according to the present invention;
- Fig. 8 -: shows a sequence of projection images acquired by the extraoral x-ray system according to the present invention;
- Fig. 9 -: shows the course of real positions of a marker throughout the sequence of projections images, with respect to a target position relative to the ceph sensor.

The reference numbers shown in the drawings designate the elements listed below, which are referred to in the following description of the exemplary embodiments.
- 1.: Extraoral x-ray system
- 2.: X-ray source
- 3.: Primary x-ray detector (PAN/DVT detector)
- 4.: Primary collimator (dashed lines)
- 5.: Cantilever arm
- 6.: Secondary x-ray detector (CEPH detector)
- 7.: Predetermined Area (Frame)
- 8.: Secondary collimator (CEPH collimator)
- 9.: Edge
- 10.: Ceph aperture
- 11.: Marker
- 12.: Recess
- 13.: Protrusion
- 14.: Hole
- 15.: Operating unit (user interface)
- 16. 16': Head fixation
- 17.: Bite block
- 18.: Computing unit (Computer)
- 19.: Display device (Screen)
- 20.: Input device (Keyboard)
- 21.: Input device (Mouse)
- 22.: X-ray projection images
- 23. 23': Cephalometric image

- A:: Position of the Patient' head for CEPH imaging
- B:: Position of the Patient's head for PAN/DVT imaging
- X:: Arrow indicating spatial adjustment to reduce chatter mark

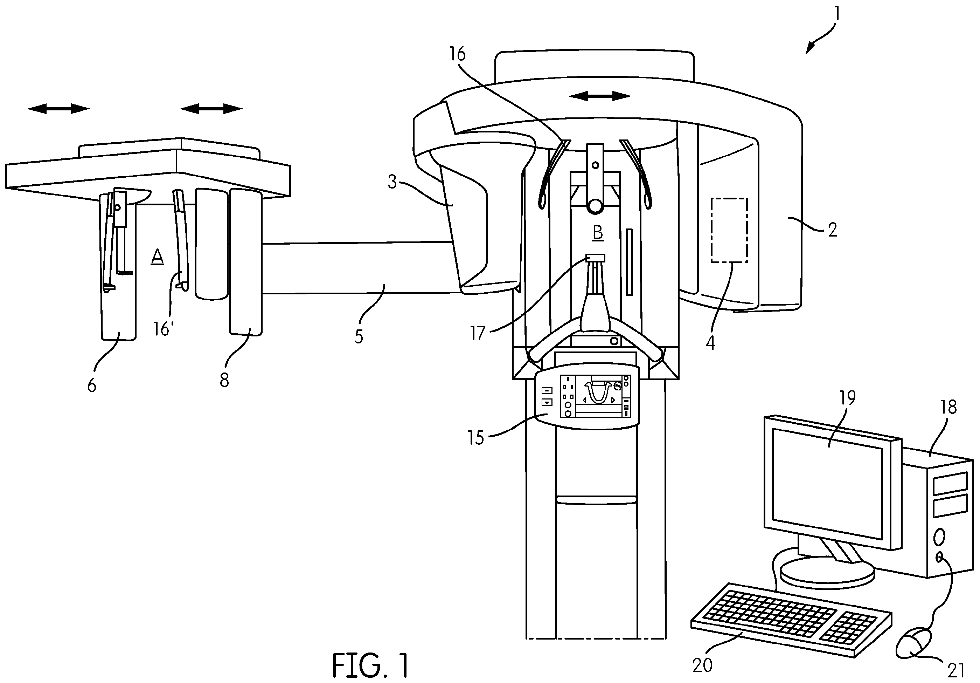

The method according to the present invention, which is explained in more detail below, is a computer-implemented method (i.e., herein also referred to as a software) and can be carried out on a computer-implemented extraoral x-ray system (1) as shown in an embodiment in Fig. 1.

The present invention also includes a corresponding computer program (i.e., the software) having computer-readable code for implementing the method. The computer program is provided on a computer readable storage medium accessible to the extraoral x-ray system (1).

The computerized extraoral x-ray system (1) shown in Fig. 1 is a multifunctional system for panoramic imaging, digital volume tomographic imaging, and cephalometric imaging. The panoramic imaging and digital volume tomographic imaging are optional. The extraoral x-ray system (1) may also be provided solely for cephalometric imaging.

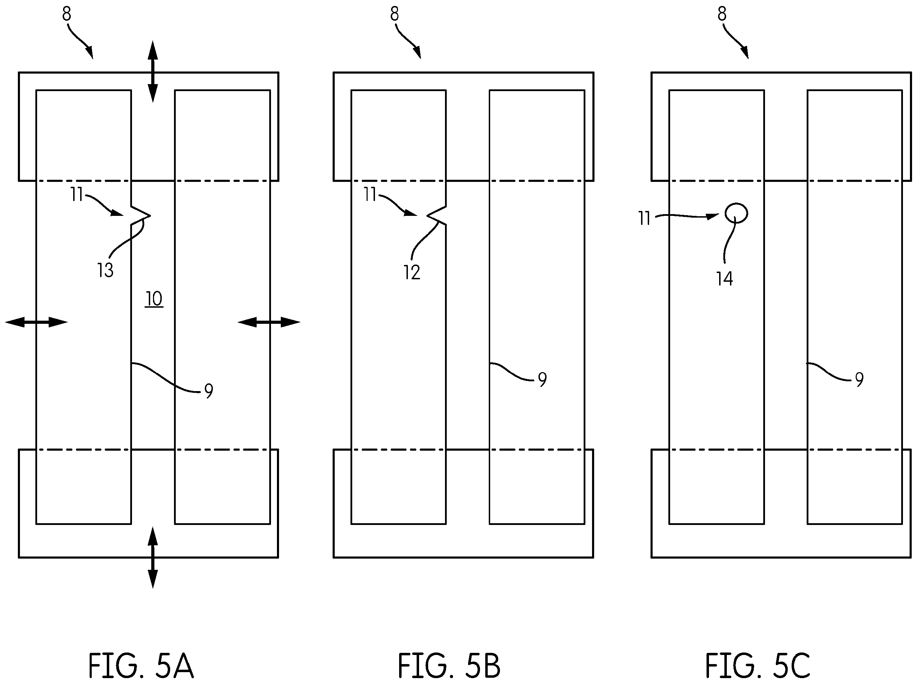

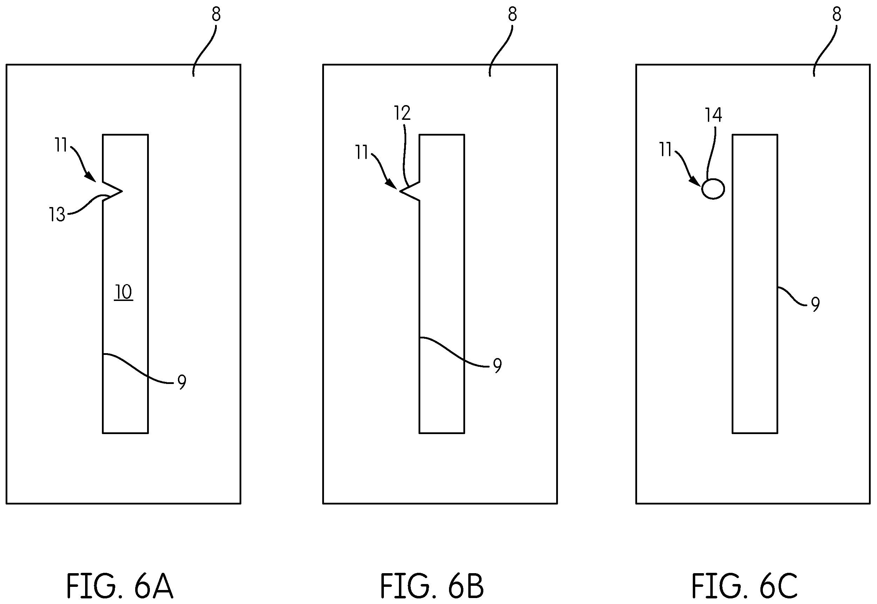

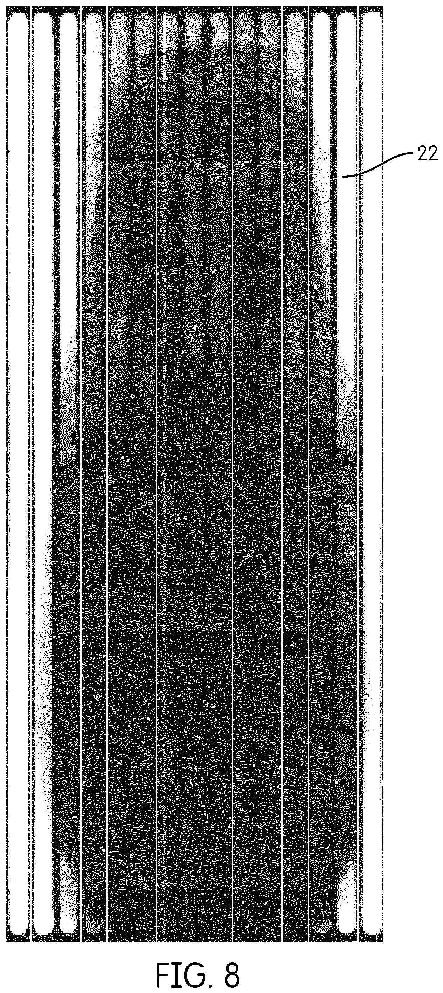

The extraoral x-ray system (1) comprises an x-ray source (2) and a primary x-ray detector (3) that are rotatably arranged around the patient's head for PAN/DVT imaging (see position B in Fig. 1). The x-ray source (2) has a primary collimator (4). The extraoral x-ray system (1) also has a cantilever arm (5) that holds a secondary x-ray detector (6) (i.e., the ceph detector) and a secondary collimator (8) (i.e., the ceph collimator). Preferably a CMOS-based ceph detector is used. This CMOS-based ceph detector is preferably a detector with a scintillator or a direct converting detector. The x-ray ceph detector (6) and the x-ray ceph collimator (8) can be linearly moved along the patient's head (see position A in Fig. 1). As shown in Fig. 8, a sequence of projection images (22) of the patient's head can be obtained by exposing the patient's head with x-rays from the x-ray source (2) while linearly moving the x-ray ceph detector (6) and the x-ray ceph collimator (8) on opposite sides along the patient's head, while the head being positioned between them. See Position A in Fig. 1 for the patient's head in the ceph mode. In the ceph mode, the primary x-ray detector (3) is automatically moved out of the x-ray beam path to a side position to avoid blocking the x-rays that are emitted from the x-ray source (2) towards the ceph collimator (8). The patient's head can be positioned in the x-ray ceph system (1) with a head fixation (16'). The x-ray ceph collimator (6) can be provided as single component structure as shown in Fig. 6 or as an adjustable (motorized) multicomponent structure as shown in Fig. 5, wherein at least the vertically extending components can be moved sideways by a motorized mechanism. Said components are made from x-ray opaque material. The x-ray ceph collimator (6) has a ceph aperture (10) and one or more x-ray opaque markers (11) which can be detected in the projection images (22). The x-ray opaque markers (11) will be explained later in more detail.

In the PAN/DVT mode, the trajectory of the x-ray source (2) and the x-ray detector (3) during the PAN/DVT imaging can describe a circular path. However, it can also assume a form deviating from this. If several actuators (not shown) are controlled simultaneously, a trajectory deviating from a pure circular path around the patient's head can be achieved. The patient's head can be positioned with a bite block (17) and optionally with a head fixation (16). See Position B in Fig. 1 for the patient's head in the PAN/DVT mode. The trajectory (*course*) of the x-ray source (2) and the primary x-ray detector (3) with respect to the bite block (17) and the head fixation (16) are known by the system (1). The primary x-ray detector (3) detects the x-rays emitted by the x-ray source (2) during the rotation. The x-ray projection images for PAN/DVT imaging are acquired, namely read out from the x-ray detector (3). The Primary x-ray detector (PAN/DVT detector) has a separate PAN detector and a separate DVT detector which can be rotated around the vertical axis to face the x-ray source (2) in accordance with the PAN/DVT imaging respectively. Or it could be a single flat panel detector capable for PAN as well as DVT.

The computerized extraoral x-ray system (1) also comprises: an operating unit (15) such as a user interface for controlling all functionalities of the modes; a computing unit (18) e.g., a computer; and a display device (19) such as a screen for visualizing any data sets (projections images and the like) resulting from the software. The CEPH/PAN/DVT modes can be each selected by the user e.g., through the operating unit (5) and/or the computer unit (18). The computer may be connected to the extraoral x-ray system (1) via a local area network (not shown) or, alternatively, via the Internet. The computer is connected to input devices such as a keyboard (20), mouse (21), and the like. The computer may be also part of a cloud. Alternatively, the computer may be integrated into the extraoral x-ray system (1). Alternatively, all or some of the computations may take place in the cloud as cloud computing or on an FPGA (field programmable gate array) in hardware. The computer executes the computer program and provides the data sets, e.g., the cephalometric images, the panoramic images, and/or the DVT images for visualization on the screen. The screen may be provided spatially separate from or integrated with the extraoral x-ray system (1). Preferably, the computer may also control all functions of the extraoral x-ray system (1). Alternatively, separate computers may be used for the control, operation, and image reconstruction.

The extraoral x-ray system (1) is preferably configurable as an IoT system, and can be bidirectionally connected via a local area network and/or the internet (not shown) to other dental devices (not shown) such as optical intraoral scanners, dental milling machines, additive manufacturing machines, and the like for cloud computing, data exchange, remote control, and the like.

The extraoral x-ray system (1) has a control means for energizing the x-ray source (2), and linearly moving the x-ray ceph detector (6), and the x-ray ceph collimator (8).

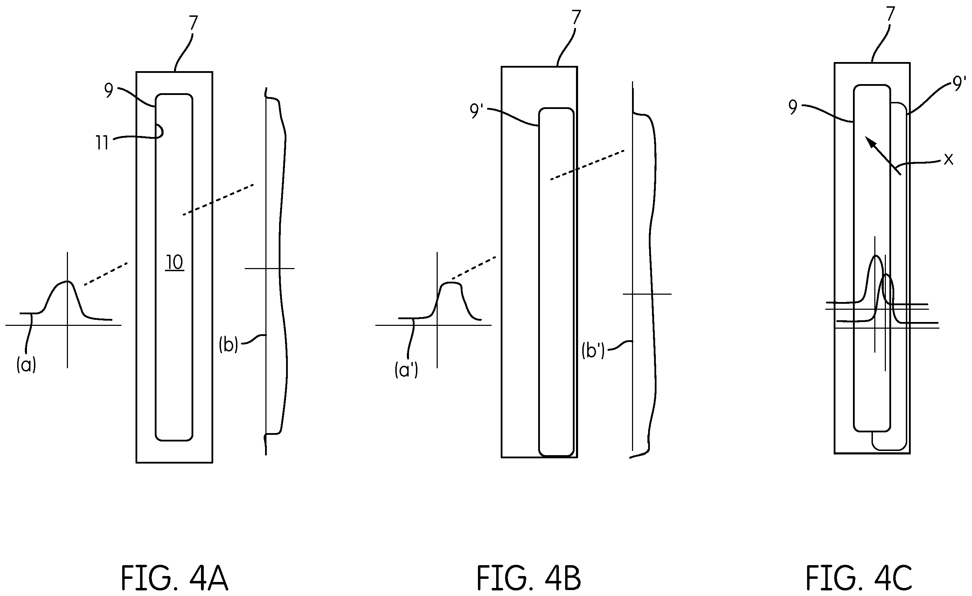

In the subsequent description, the computer-implemented method for generating a cephalometric image (23) of a patient's head will be explained in more detail. The extraoral x-ray system (1) has a control means configured to execute the method. The method comprises the following steps:
In an initial step, a sequence of projection images (22) is acquired by exposing the patient's head with x-rays from the x-ray source (2) while linearly moving the x-ray ceph detector (6) and the x-ray ceph collimator (8) along the patient's head. The projection images (20) are sequentially read out from a predetermined frame (7) (see Fig. 4) of the x-ray ceph detector (6). The frame-based ceph detector (6) has a constant frame rate preferably e.g., 300fps-500fps (fps: frames per second). The projection images (22) are preferably pre-processed for dark current, gain and pixel defects. The x-ray ceph collimator (8) has an aperture (10) and one or more x-ray opaque markers (11) which can be detected in the projection images (22) or the pre-processed projection images (22). In the subsequent description the projection images (22) may also refer to preprocessed projection images (22) for the sake of comprehensibility and ease of wording.

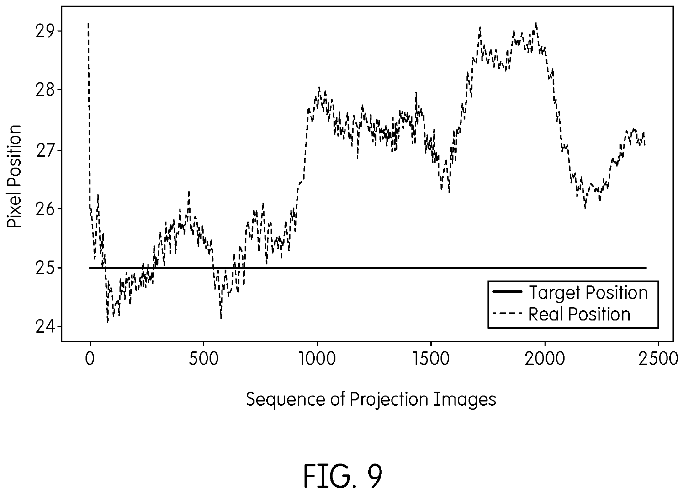

In a further step, the real position of the at least one x-ray opaque marker (11) is detected in each projection images (22) relative to a target position on the ceph detector (6). The target position on the ceph detector (6) is ideally known by the system (1). Fig. 9 shows an example in which the real position of the marker (11) (dashed curve) on the ceph detector (6) is detected for the sequence of projection images (22) e.g., by image processing. The solid horizontal line in Fig. 9 shows the target position on the ceph detector (6).

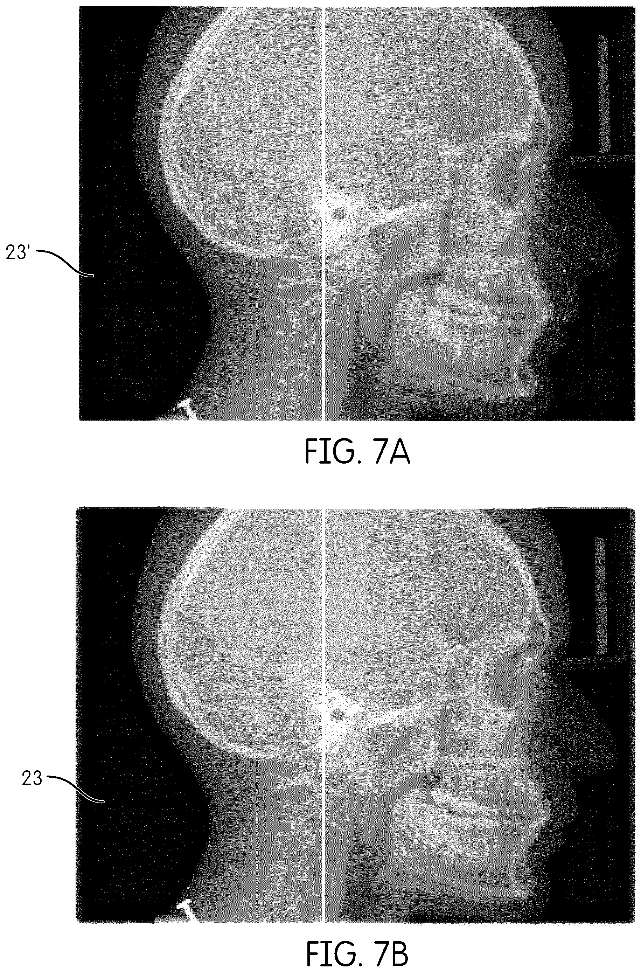

In a further shift & add reconstruction step, the projection images (22) are added after shifting each projection image (22) by a predetermined shift amount relative to its preceding projection image (22) to obtain the cephalometric image (23) as shown in Fig. 7B. The predetermined shift amount can be set based on the frame rate used, and the linear speed set for the ceph detector (6). In the ideal case no adjustment to the projection images (22) are necessary when the ceph aperture (10), consequently the image data distribution is centered with respect to the predetermined frame (7) throughout the sequence. Fig. 4a shows an ideal case where the ceph collimator (8) is correctly positioned on the predetermined frame (7) i.e., centered. Fig. 4b shows a case where the ceph collimator (8) is incorrectly positioned on the predetermined frame (7). In such case, the image data grey value distribution (see e.g., the graphical representations) is offset from the center of the frame (7). The graphical representations (a) and (a') show horizontal cross sections of the image data grey value distribution. The graphical representations (b) and (b') show vertical cross sections of the image data grey value distribution. The boundary of the distribution can be set by threshold grey values. However, to reduce chatter marks, before the adding procedure, the positions of the shifted projection images (22) can be individually adjusted by an amount corresponding to a difference in the real position and the target position of the least one marker (11) detected in the corresponding projection image (22). Fig. 4c shows an arrow X indicating the adjustment to be made to reduce the mispositioning i.e., to coincide the grey value distribution of the image data symmetrically with respect to the midpoint of the frame. The arrow X links the real position of the detected marker (11) to the target position of the marker (11), in this example the edge (9) serves as the marker (11). The shifted projection image (22) can be adjusted by calculating an individual 2 by 2 matrix matching the predetermined frame size (7) based on the direction and length of the arrow X, and then by transforming (i.e., shifting) the projection image (22) from the real position to the target position by means of the said individual matrix. Herein the matrix is used to map the image data from the real position to the target position. The size of the matrix matches the pixels in the column and rows of the frame (7). After the shifted projection images (20) are individually adjusted, these are added in the shift & add reconstruction step to obtain the cephalometric image (23) as shown in Fig. 7B. For comparison, Fig. 7A shows a cephalometric image (23') in which the shifted projection images (20) have not been adjusted. The mapping of the image data prevents the asymmetrical grey value distributions to become conspicuous in form of chatter marks i.e., grey value stripes. The mapping is generally at the order of one pixel or a few pixels. The mapping can be also implemented with sub pixel accuracy, however combined with interpolation methods. Therefore, the mapping reduces chatter marks at the cost of introducing a negligible motion artifact which is, however, not conspicuous to the dentist's eye.

In the subsequent description the various markers (11) will be explained in more detail.

In a preferred embodiment, the marker (11) is defined through the ceph collimator edge (9) surrounding the aperture (10) (see e.g., Fig. 4A). For example, the corner of the ceph collimator edge (9) or a location at predetermined distance to the corner can serve as a marker (11). The marker (11) is thus visual in the image data of the projection images (22), for example, through the deflection point of the grey scale values of the image data, and can be located by image processing. Herein the edge (9) extend ideally along the horizontal and vertical directions.

In another preferred embodiment, as shown in Fig. 5A-5B and Fig. 6A-6B, the marker (11) comprises a recess (12) or a protrusion (13) formed on the ceph collimator edge (9) surrounding the aperture (10). The recess/protrusion shape of the marker (11) will be visual in the image data of the projection images (22), for example, through the grey scale values of the image data, and can be located by image processing. More than one recess (12) and/or protrusion (13) can be formed in the radio opaque material of the edge (9).

In another preferred embodiment, as shown in Fig. 5C and Fig. 6C, the marker (11) comprises a hole (14) formed in the vicinity of the ceph collimator edge (9) surrounding the aperture (10). More than one hole (14) can be formed. The hole shape of the marker (11) will be visual in the image data of the projection images (22), for example, through the grey scale values of the image data, and can be located by image processing.

The recesses (12), protrusions (13), holes (14), and edges (9) can be used either singly or in combination as the markers (11).

In a further embodiment, the above-described adjustment is further extended to deal with skew alignment of the x-ray ceph collimator (8) with respect to the ceph detector (6). Therefore, in the detecting step the real position of at least one additional second x-ray opaque marker (11) is detected in each of the projection images (22) relative to a target position on the ceph detector (6) to determine the alignment to be adjusted. Therewith two arrows X' (not shown) can be derived for the two markers (11). Subsequently, based on the two arrows X', an individual matrix can be derived which involves translation and rotation for each projection image (22). Thereby, the alignment of the shifted projection images (22) can be individually adjusted in accordance with the two markers (11) detected in the corresponding projection (22) image prior to the adding.

## Claims

1. A computer-implemented method of generating a cephalometric image (23) of a patient's head, comprising:
a step of acquiring a sequence of projection images (22) by exposing the patient's head with x-rays from an x-ray source (2) while linearly moving an x-ray ceph detector (6) and an x-ray ceph collimator (8) on opposing sides along the patient's head, wherein the x-ray ceph collimator (8) has an aperture (10) and one or more x-ray opaque markers (11) which can be detected in the projection images (22), and wherein the projection images (22) are sequentially read out at a predetermined rate from a predetermined frame (7) of the x-ray ceph detector (6);
a step of detecting the real position of the at least one x-ray opaque marker (11) in each of the projection images (22) relative to a target position on the ceph detector (6);
a shift and add reconstruction step of adding the projection images (20) after shifting each projection image (22) relative to its preceding projection image (22) by a predetermined shift amount based on the predetermined rate;
wherein the positions of the shifted projection images (22) are individually adjusted by an amount corresponding to a difference in the real position and the target position of the least one marker (11) detected in the corresponding projection image (22) to reduce chatter marks prior to the adding in the shift and add reconstruction step.

2. The computer-implemented method according to claim 1, **characterized in that**
the marker (11) is defined through the ceph collimator edge (9) surrounding aperture (10).

3. The computer-implemented method according to claim 1 or 2, **characterized in that**
the marker (11) comprises one or more recesses (12) and/or protrusions (13) formed on the ceph collimator edge (9) surrounding the aperture (10).

4. The computer-implemented method according to any one of claims 1 to 3, **characterized in that** the marker (11) comprises one or more holes (14) formed in the vicinity of the ceph collimator edge (9) surrounding the aperture (10).

5. The computer implemented method according to any one of claims 1 to 4, **characterized in that**
in the detecting step the real position of at least one additional x-ray opaque marker (11) is detected in each of the projection images (22) relative to a corresponding target position on the ceph detector (6) in order to determine the alignment to be adjusted,
wherein the alignment of the shifted projection images (22) are also individually adjusted in accordance with the two markers (11) detected in the corresponding projection (22) image prior to the adding.

6. An extraoral x-ray system (1), comprising:
an x-ray source (2); an x-ray ceph detector (6); and an x-ray ceph collimator (8), wherein the x-ray ceph collimator (8) has a x-ray opaque marker (11);
a control means for controlling the x-ray source (2), the x-ray ceph detector (6) and the x-ray ceph collimator (8);
wherein the control means is configured to execute the method according to any one of the preceding claims.

7. A computer program comprising computer-readable code which, when executed by a computer-assisted extraoral x-ray system (1), causes said system (1) to perform the method of any one of claims 1 to 5.

## Patentansprüche

1. Computerimplementiertes Verfahren zur Erzeugung eines zephalometrischen Bildes (23) des Kopfes eines Patienten, umfassend:
einen Schritt des Erfassens einer Sequenz von Projektionsbildern (22) durch Bestrahlen des Kopfes des Patienten mit Röntgenstrahlen von einer Röntgenquelle (2), während ein Röntgen-Zeph-Detektor (6) und ein Röntgen-Zeph-Kollimator (8) auf gegenüberliegenden Seiten entlang des Kopfes des Patienten linear bewegt werden, wobei der Röntgen-Zeph-Kollimator (8) eine Apertur (10) und eine oder mehrere röntgenopake Markierungen (11) aufweist, die in den Projektionsbildern (22) detektiert werden können, und wobei die Projektionsbilder (22) sequentiell mit einer vorbestimmten Rate von einem vorbestimmten Rahmen (7) des Röntgen-Zeph-Detektors (6) ausgelesen werden;
einen Schritt des Detektierens der tatsächlichen Position der zumindest einen röntgenopaken Markierung (11) in jedem der Projektionsbilder (22) relativ zu einer Zielposition auf dem Zeph-Detektor (6);
einen Verschieben-und-Addieren-Rekonstruktionsschritt des Addierens der Projektionsbilder (20) nach dem Verschieben jedes Projektionsbildes (22) relativ zu seinem vorhergehenden Projektionsbild (22) um eine vorbestimmte Verschiebungsmenge basierend auf der vorbestimmten Rate;
wobei die Positionen der verschobenen Projektionsbilder (22) individuell um eine Menge entsprechend einer Differenz der tatsächlichen Position und der Zielposition der zumindest einen Markierung (11), die in dem entsprechenden Projektionsbild (22) detektiert wird, angepasst werden, um Rattermarken vor dem Addieren in dem Verschieben-und-Addieren-Rekonstruktionsschritt zu reduzieren.

2. Computerimplementiertes Verfahren nach Anspruch 1, **dadurch gekennzeichnet, dass** die Markierung (11) durch die Zeph-Kollimatorkante (9), welche die Apertur (10) umgibt, definiert ist.

3. Computerimplementiertes Verfahren nach Anspruch 1 oder 2, **dadurch gekennzeichnet, dass** die Markierung (11) eine oder mehrere Ausnehmungen (12) und/oder Vorsprünge (13) umfasst, die an der Zeph-Kollimatorkante (9), welche die Apertur (10) umgibt, gebildet sind.

4. Computerimplementiertes Verfahren nach einem der Ansprüche 1 bis 3, **dadurch gekennzeichnet, dass** die Markierung (11) ein oder mehrere Löcher (14) umfasst, die in der Nähe der Zeph-Kollimatorkante (9), welche die Apertur (10) umgibt, gebildet sind.

5. Computerimplementiertes Verfahren nach einem der Ansprüche 1 bis 4, **dadurch gekennzeichnet, dass**
in dem Detektierungsschritt die tatsächliche Position von zumindest einer zusätzlichen röntgenopaken Markierung (11) in jedem der Projektionsbilder (22) relativ zu einer entsprechenden Zielposition auf dem Zeph-Detektor (6) detektiert wird, um die anzupassende Ausrichtung zu bestimmen,
wobei die Ausrichtung der verschobenen Projektionsbilder (22) gemäß den zwei Markierungen (11), die in dem entsprechenden Projektionsbild (22) detektiert werden, vor dem Addieren auch individuell angepasst werden.

6. Extraorales Röntgensystem (1), umfassend:
eine Röntgenquelle (2); einen Röntgen-Zeph-Detektor (6); und einen Röntgen-Zeph-Kollimator (8), wobei der Röntgen-Zeph-Kollimator (8) eine röntgenopake Markierung (11) aufweist;
ein Steuermittel zum Steuern der Röntgenquelle (2), des Röntgen-Zeph-Detektors (6) und des Röntgen-Zeph-Kollimators (8);
wobei das Steuermittel dazu konfiguriert ist, das Verfahren nach einem der vorhergehenden Ansprüche auszuführen.

7. Computerprogramm, das computerlesbaren Code umfasst, der, wenn durch ein computergestütztes extraorales Röntgensystem (1) ausgeführt, bewirkt, dass das System (1) das Verfahren nach einem der Ansprüche 1 bis 5 durchführt.

## Revendications

1. Procédé mis en œuvre par ordinateur permettant de générer une image céphalométrique (23) de la tête d'un patient, comprenant :
une étape d'acquisition d'une séquence d'images de projection (22) en exposant la tête du patient à des rayons X provenant d'une source de rayons X (2) tout en déplaçant linéairement un détecteur céphalométrique à rayons X (6) et un collimateur céphalométrique à rayons X (8) sur des côtés opposés le long de la tête du patient, ledit collimateur céphalométrique à rayons X (8) possédant une ouverture (10) et un ou plusieurs marqueurs opaques aux rayons X (11) qui peuvent être détectés dans les images de projection (22), et lesdites images de projection (22) étant lues séquentiellement à une vitesse prédéfinie à partir d'une image prédéfinie (7) du détecteur céphalométrique à rayons X (6) ;
une étape de détection de la position réelle dudit au moins un marqueur opaque aux rayons X (11) dans chacune des images de projection (22) par rapport à une position cible sur le détecteur céphalométrique (6) ;
une étape de reconstruction par décalage et ajout d'ajout des images de projection (20) après le décalage de chaque image de projection (22) par rapport à son image de projection précédente (22) d'une quantité de décalage prédéfinie sur la base de la vitesse prédéfinie ;
lesdites positions des images de projection décalées (22) étant ajustées individuellement d'une quantité correspondant à une différence entre la position réelle et la position cible dudit au moins un marqueur (11) détectées dans l'image de projection correspondante (22) de manière à réduire les marques de broutement avant l'ajout dans l'étape de reconstruction par décalage et ajout.

2. Procédé mis en œuvre par ordinateur selon la revendication 1, **caractérisé en ce que**
le marqueur (11) est défini à travers le bord du collimateur céphalométrique (9) entourant l'ouverture (10).

3. Procédé mis en œuvre par ordinateur selon la revendication 1 ou 2, **caractérisé en ce que**
le marqueur (11) comprend un ou plusieurs évidements (12) et/ou saillies (13) formés sur le bord du collimateur céphalique (9) entourant l'ouverture (10).

4. Procédé mis en œuvre par ordinateur selon l'une quelconque des revendications 1 à 3, **caractérisé en ce que** le marqueur (11) comprend un ou plusieurs trous (14) formés à proximité du bord du collimateur céphalométrique (9) entourant l'ouverture (10).

5. Procédé mis en œuvre par ordinateur selon l'une quelconque des revendications 1 à 4, **caractérisé en ce que**
dans l'étape de détection, la position réelle d'au moins un marqueur opaque aux rayons X supplémentaire (11) est détectée dans chacune des images de projection (22) par rapport à une position cible correspondante sur le détecteur céphalométrique (6) afin de déterminer l'alignement devant être ajusté,
ledit alignement des images de projection décalées (22) étant également ajusté individuellement conformément aux deux marqueurs (11) détectés dans l'image de projection correspondante (22) avant l'ajout.

6. Système à rayons X extra-buccal (1), comprenant :
une source de rayons X (2) ; un détecteur céphalométrique à rayons X (6) ; et un collimateur céphalométrique à rayons X (8), ledit collimateur céphalométrique à rayons X (8) possédant un marqueur opaque aux rayons X (11) ;
un moyen de commande destiné à commander la source de rayons X (2), le détecteur céphalométrique à rayons X (6) et le collimateur céphalométrique à rayons X (8) ;
ledit moyen de commande étant configuré pour exécuter le procédé selon l'une quelconque des revendications précédentes.

7. Programme informatique comprenant un code lisible par ordinateur qui, lorsqu'il est exécuté par un système à rayons X extra-buccal assisté par ordinateur (1), amène ledit système (1) à exécuter le procédé selon l'une quelconque des revendications 1 à 5.
